# EUROPÄISCHE PATENTSCHRIFT

(11) **EP 0 857 043 B1**
(45) Veröffentlichungstag und Bekanntmachung des Hinweises auf die Patenterteilung: **08.08.2001**
(21) Anmeldenummer: 96931714.8
(22) Anmeldetag: 03.10.1996
(51) Int. Cl.: A61F 2/44

(54) **ZWISCHENWIRBEL-IMPLANTAT**
INTER-VERTEBRAL IMPLANT
IMPLANT INTERVERTEBRAL

(30) Priorität: 20.10.1995 WO PCT/CH95/00245
(43) Veröffentlichungstag der Anmeldung: 12.08.1998
(73) Patentinhaber: SYNTHES AG Chur, 7002 Chur (CH)
(72) Erfinder: COTTLE, William, Vancouver, British Columbia V6S 1G3 (CA)
(74) Vertreter: Lusuardi, Werther Giovanni, Dr.
(86) Internationale Anmeldenummer: CH9600346
(87) Internationale Veröffentlichungsnummer: WO9715248

(56) Entgegenhaltungen:
- EP-A- 0 307 241
- EP-A- 0 646 366
- WO-A-95/08306
- WO-A-95/08964
- WO-A-95/32673
- US-A- 5 192 327
- US-A- 5 294 391

## Beschreibung

Die Erfindung betrifft ein Zwischenwirbel-Implantat gemäss dem Oberbegriff des Patentanspruchs 1 sowie eine Kombinationsimplantat gemäss dem Oberbegriff des Anspruchs 21.

Solche Zwischenwirbel-Implantate werden bei der Fusion von zwei Wirbelkörpern eingesetzt, insbesondere im Bereich der lumbalen Wirbelsäule. Pro Zwischenwirbelraum werden ein oder zwei Implantate verwendet.

Aus dem Stand der Technik sind bereits verschiedene Typen derartiger Zwischenwirbel-Implantate bekannt, welche allerdings den Nachteile aufweisen, dass sie die Gefahr des Einsinkens der Implantate in die Endplatten der betroffenen Wirbel beinhalten. Beispielsweise ist aus der US 5,192,327 BRANTIGAN ein Zwischenwirbel-Implantat in Form eines oben und unten offenen Ringes oder Doppelringes bekannt. Da nur der Rand des Ring-Implantates und allenfalls noch der schmale Verbindungssteg bei einer Doppelring-Ausführung als Knochenkontaktfläche in Frage kommt, ist die Gefahr des Einsinkens der Endplatten der damit beabstandeten Wirbelkörper ziemlich gross.

Die WO-A-9508306 zeigt ein weiteres Zwischenwirbel-Implantat in Form eines oben und unten offenen Käfigs, welches die gleichen Nachteile aufweist wie die US 5,192,327.

Hier will die Erfindung Abhilfe schaffen. Die Erfindung löst die Aufgabe, ein Zwischenwirbel-Implantat zu schaffen, welches in kontrollierbarer Weise in den Zwischenwirbelraum einführbar ist, eine optimale Knochenkontaktfläche aufweist und dank einer Anzahl von Perforationen in der Knochenkontaktfläche trotzdem ein gutes Einwachsverhalten des Knochens zeigt.

Zur Lösung dieser Aufgabe ist die eingangs genannte Anordnung durch die Merkmale des kennzeichnenden Teils des unabhängigen Anspruchs 1 weitergebildet.

Damit ist der Vorteil erzielbar, dass dank der grossen Knochen-Auflagefläche der Deck- und Grundfläche ein Einsinken des Implantats in die Endplatten der Wirbelkörper verhindert wird.
Gleichzeitig gestatten jedoch eine Anzahl von Perforationen in der Deck- und/oder Grundfläche das Einwachsen des Knochens. Die Perforationen in der Deck- und/oder Grundfläche sind für das Einwachsen des Knochens, welche die Fusion der benachbarten Wirbelkörper bewirkt, von grösster Wichtigkeit. Es hat sich überraschenderweise gezeigt, dass die geometrischen Verhältnisse dieser Perforationen für den klinischen Erfolg von ausschlaggebender Bedeutung sind. Wenn die Gesamtfläche dieser Perforationen zu klein ist kann der Knochen nicht im erforderlichen Mass einwachsen, so dass keine Fusion eintritt. Ist anderseits die Gesamtfläche dieser Perforationen zu gross, so ist die verbleibende Kontaktfläche der Deck- und Grundfläche des Implantats zu den Endplatten der benachbarten Wirbelkörper zu klein, was zu hohen Kontaktkräften zwischen Implantat und Endplatten führt, die wiederum die Gefahr eines Einsinkens des Implantats in die Endplatten erhöht.
Es hat sich gezeigt, dass die Gesamtfläche der Perforationen in der Deck- und/oder Grundfläche im Bereich von 40 bis 55 % der Gesamtfläche Deck- und/oder Grundfläche betragen muss, um gute klinische Resultate zu erzielen. Vorzugsweise sollte die Gesamtfläche der Perforationen in der Deck- und/oder Grundfläche 43 bis 51 %, typischerweise 45 - 49 % der Gesamtfläche der Deck- und/oder Grundfläche betragen.

Von besonderer Bedeutung für den zu erzielenden klinischen Erfolg haben sich auch die Dimensionen der einzelnen Perforationen in der Deck- und/oder Grundfläche erwiesen. Falls die Fläche der einzelnen Perforationen zu klein ist, wird das Einwachsen des Knochens erschwert, auch wenn insgesamt die Perforationsfläche erheblich sein kann. Anderseits wirken auch Perforationen in der Deck- und/oder Grundfläche mit zu grosser Durchtritts-Fläche in negativer Weise, indem sie die gleichmässige Abstützung der Endplatte beinträchtigen, so dass die Gefahr eines lokales Einsinken des Implantats in die Endplatte besteht. Es hat sich gezeigt, dass die Einzelfläche einer einzelnen Perforation höchstens 20 % der Gesamtfläche der Deck- und/oder Grundfläche betragen darf, um gute klinische Resultate zu erzielen. Vorzugsweise sollte die Einzelfläche einer einzelnen Perforation 5 - 15 %, typischerweise 8 - 13 % der Gesamtfläche der Deck- und/oder Grundfläche betragen.

Der Durchmesser der Perforationen sollte vorzugsweise höchstens 9,0 mm, typischerweise höchstens 5,0 mm betragen. Die im Randbereich der Deck-- und/oder Grundfläche angebrachten Perforationen sollten durchschnittlich kleiner sein als die im Zentralbereich der Deck- und/oder Grundfläche angebrachten Perforationen, vorzugsweise mit einer graduellen Zunahme des Durchmesser von aussen nach innen. Damit wird erreicht, dass die zentral angebrachten Perforationen das Einwachsen des Knochens an der dünnsten - am besten geeigneten - Stelle der Endplatte erlauben, währenddem anderseits die periphere Partie Deck- und/oder Grundfläche die beste Kontaktfläche zur dichteren Randpartie der knöchernen Endplatte ergibt.

Schliesslich hat sich auch gezeigt, dass die geometrischen Verhältnisse des als Hohlkörper ausgebildeten Implantats für den klinischen Erfolg wichtig sind. Um eine gute Fusion der benachbarten Wirbel erzielen zu können ist es notwendig das Verhältnis VH/VK zwischen dem Volumen des Hohlraums VH und dem Gesamtvolumen VK des Käfigs in einem hohen Bereich von 70 - 90 % zu halten. Damit ist die leichte Einführung von Knochenspänen oder Knochenersatzmaterialien garantiert, welche erste optimale Voraussetzungen für die Fusion bietet. Vorzugsweise sollte das Verhältnis VH/VK zwischen Volumen des Hohlraums VH und Gesamtvolumen VK des Käfigs im Bereich von 75 - 85 %, typischerweise von 78 - 82 % liegen.

Bei einer bevorzugten Ausführungsform mit einer dreidimensionalen Strukturierung der Deck- und Grundfläche des Käfigs wird zusätzlich eine hohe Lagestabilität des Implantates erreicht wird. Die dreidimensionale Strukturierung kann aus Zähnen, Längsnuten oder anderen geeigneten Erhebungen oder Vertiefungen bestehen. Die Höhe dieser Strukturierung sollte 0,5 - 2,0 mm, vorzugsweise von 1,0 - 1,5 mm betragen und kann beispielsweise aus Zähnen, vorzugsweise in regelmässiger Anordnung, bestehen.
Die dreidimensionalen Strukturierung kann eine strukturierte Hydroxylapatitbeschichtung sein. Es ist auch möglich, den gesamten Käfig mit Hydroxylapatit oder einem anderen bioaktiven Material zu beschichten.
Die dreidimensionalen Strukturierung kann aber auch eine strukturierte Beschichtung aus Titan, Titanlegierungen oder anderen körperverträglichen Metallen sein.
Vorzugsweise weisen die Deck- und Grundflächen einen freien Rand ohne Strukturierung auf.

Bei einer weiteren Ausführungsform ist die Deck- und Grundfläche nach aussen konvex gewölbt ausgebildet, um eine optimale Anpassung an die Geometrie der Endplatten der benachbarten Endplatten der Wirbelkörper zu erzielen.

Bei einer weiteren Ausführungsform sind auch die Seitenflächen mit Perforationen versehen, deren Gesamtfläche höchstens 40 % (typischerweise höchstens 30 %) und mindestens 15 % (typischerweise mindestens 20 %) der Gesamtfläche der Seitenflächen betragen sollten. Die Perforationen in den Seitenflächen sind vorzugsweise Langlochausnehmungen.
Auch die Frontwand kann mit Perforationen, vorzugsweise in Form von Längsausnehmungen, versehen sein.
Bei einer weiteren Ausführungsform weist die Frontwand Mittel zur Aufnahme eines Instrumentes auf, mit welchem der Käfig manipuliert werden kann. Auch die Seitenflächen können Mittel zur Aufnahme eines Instrumentes aufweisen, mit welchem der Käfig manipuliert werden kann.

Bei einer weiteren Ausführungsform der Erfindung werden zwei Zwischenwirbel-Implantaten zu einem Kombinationsimplantat vereinigt, wobei die beiden Zwischenwirbel-Implantate an ihren fehlenden Seitenflächen einstückig miteinander verbunden sind. Vorzugsweise weist die kombinierte Frontwand eine Langlochausnehmung auf.

Gegenüber dem Stand der Technik ergeben sich folgende Vorteile des erfindungsgemässen Implantats
a) Verrutschsicherheit;
b) Verbesserte Röntgendurchlässigkeit:
   Dank der Perforationen in den Seitenflächen, sowie in der
   Front- und Hinterwand lässt sich das Fusionsverhalten des
   Implantats via Röntgenaufnahmen leicht kontrollieren, was bei Implantaten gemäss dem Stand der Technik mit geschlossenen Seitenflächen stark erschwert ist.
c) Kompressibilität von allfällig in den Käfig eingeführtem Knochenmaterial.

Die Erfindung und Weiterbildungen der Erfindung werden im folgenden anhand der teilweise schematischen Darstellungen einiger Ausführungsbeispiele noch näher erläutert.

### Es zeigen:

Fig. 1 eine perspektivische Darstellung des erfindungsgemässen Implantats;
Fig. 2 ein Längsschnitt durch das Implantat nach Fig. 1;
Fig. 3 ein Querschnitt durch das Implantat nach Fig. 1;
Fig. 4 eine perspektivische Darstellung einer Variation eines erfindungsgemässen Implantats;
Fig. 5 eine Aufsicht auf das Implantats nach Fig. 4;
Fig. 6 eine Seitenansicht des Implantats nach Fig. 4;
Fig. 7 eine Ansicht von der Hinterseite des Implantats nach Fig. 4; und
Fig. 8 eine Aufsicht auf ein modifiziertes Implantat nach Fig. 4.

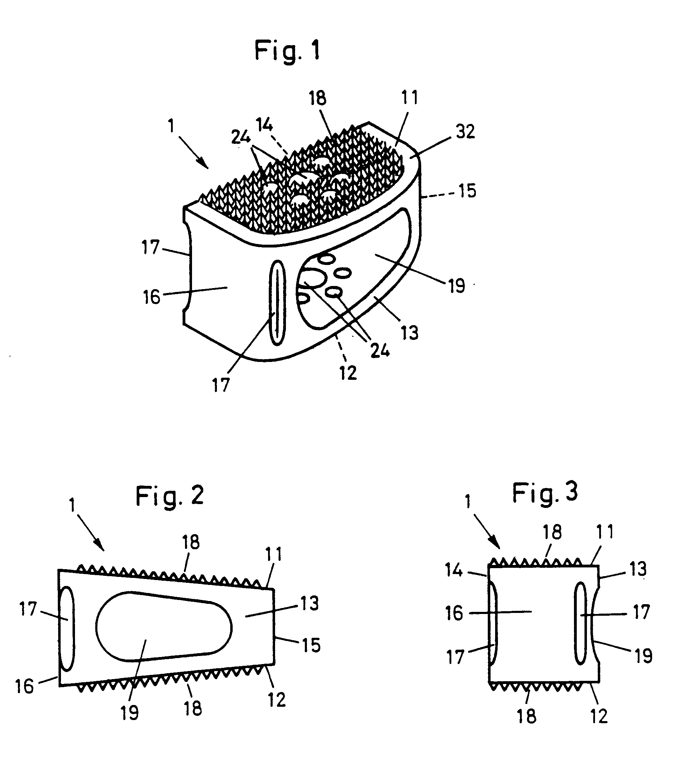

Das in den Fig. 1 - 3 dargestellte Zwischenwirbel-Implantat besteht im wesentlichen aus einem (abgesehen von Perforationen) an seiner Deckfläche 11 und Grundfläche 12 geschlossenen, rahmenförmigen Käfig 1 mit zwei je ein Langloch 19 aufweisenden Seitenflächen 13 und 14, einer zwei Nuten 17 aufweisenden Frontwand 16 und einer Hinterwand 15. Die Nuten 17 dienen der Aufnahme eines Manipulationsinstrumentes. Die Form des Käfigs 1 ist keilförmig ausgebildet, d.h. mit gegen die Frontwand 16 hin divergierenden Deck- und Grundflächen 11,12.

Die Deck- und Grundfläche 11,12 weist bei der Ausführung gemäss den Fig. 1 - 3 eine dreidimensionale Strukturierung 18 auf, vorzugsweise in Form von spitzen Zähnen in regelmässiger Anordnung mit einer Höhe von ca. 1,75 mm, um die Lagestabilität des Implantats zu verbessern. Deck- und Grundfläche 11,12 weisen einen freien Rand 32 ohne Strukturierung 18 auf. Der freie Rand 32 vermindert die Verletzungsgefahr während und nach der Operation.

Die Deck- und Grundfläche 11,12 ist mit einer Mehrzahl von Perforationen 24 versehen, deren Gesamtfläche 40 % der Gesamtfläche der Deck- und Grundfläche 11,12 beträgt. Die Einzelfläche einer einzelnen Perforation 24 beträgt 15 % der Gesamtfläche der Deck- und Grundfläche 11,12 beträgt. Das Verhältnis VH/VK zwischen Volumen VH des Hohlraums 20 und Gesamtvolumen VK des Käfigs 1 beträgt 0,22.

Die Frontwand 16 des Käfigs 1 ist - wie in Fig. 1 ersichtlich - mit zwei Nuten 17 zur Aufnahme eines Instrumentes versehen, damit der Käfig 1 in den Zwischenwirbelraum eingeführt und positioniert werden kann.

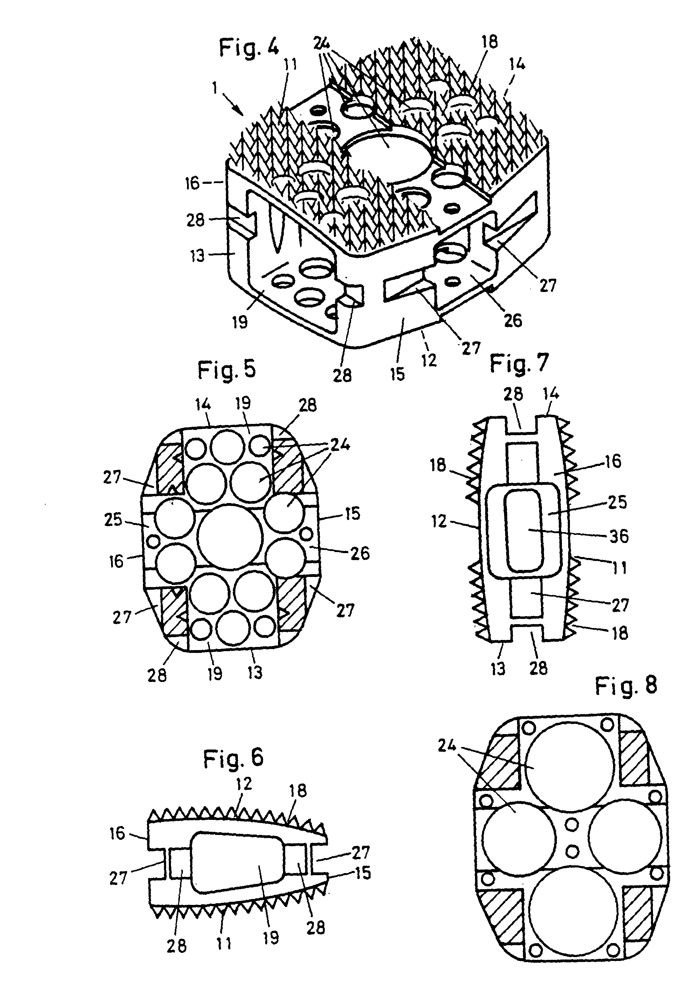

Weitere Ausführungsformen der Erfindung sind in den Fig. 4 8 dargestellt, welche abgesehen von den nachstehend beschriebenen Abänderungen die gleichen Merkmale wie die Ausführungsform gemäss den Fig. 1 - 3 aufweisen. Das erfindungsgemässe Implantat besteht aus einem rahmenförmigen Käfig 1 mit Deckfläche 11, Grundfläche 12, zwei mit je einem Langloch 19 versehenen Seitenflächen 13 und 14, einer eine Öffnung 25 aufweisenden Frontwand 16 und einer eine Öffnung 26 aufweisenden Hinterwand 15. Die Öffnung 25 weist laterale Nuten 27 auf, welche ein geeignetes Manipulationsinstrument aufnehmen können. Auch die in den Seitenfläche 13,14 positionierten Langlöcher 19 sind mit lateralen Nuten 28 versehen, welche ein geeignetes Manipulationsinstrument aufnehmen können.

Die Form des Käfigs 1 ist keilförmig ausgebildet, d.h. mit gegen die Frontwand 16 hin divergierenden Deck- und Grundflächen 11,12.
Die Deck- und Grundfläche 11,12 bei der Ausführungsform gemäss den Fig. 4 - 7 ist mit einer Mehrzahl von Perforationen 24 versehen, deren Gesamtfläche 48 % der Gesamtfläche der Deck- und Grundfläche 11,12 beträgt. Die Einzelfläche einer einzelnen Perforation 24 beträgt 10 % der Gesamtfläche der Deck- und Grundfläche 11,12 beträgt. Das Verhältnis VH/VK zwischen Volumen VH des Hohlraums 20 und Gesamtvolumen VK des Käfigs 1 beträgt 0,21.

Die Performationen 24 in den Deck- und Grundfläche 11,12 des Implantats können - innerhalb der erfindungsgemässen Bereiche - in mancherlei Hinsicht variiert werden. Fig. 8 zeigt z.B. eine Variation der Performationen 24 in den Deck- und Grundflächen 11,12 des Implantats nach Fig. 4, bei welcher die Gesamtfläche 50 % der Gesamtfläche der Deck- und Grundfläche 11,12 beträgt. Die Einzelfläche einer einzelnen Perforation 24 beträgt 15 % der Gesamtfläche der Deck- und Grundfläche 11,12. Das Verhältnis VH/VK zwischen Volumen VH des Hohlraums 20 und Gesamtvolumen VK des Käfigs 1 beträgt 0,22.

Der Käfig 1 kann bei allen Ausführungsformen aus Titan, Titanlegierung, Keramik oder einem biokompatiblen Kunststoff, z.B. Polyethylen, gefertigt werden.

Nachstehend wird nun die klinische Anwendung im Detail beschrieben.
Der in Fig. 1 gezeigte Käfig 1 wird durch die seitlichen Perforationen 19 in Form von Langlochausnehmungen mit Knochenspänen (bone graft) oder Knochenersatzmaterial, eventuell unter Komprimierung desselben, gefüllt. Danach wird der gefüllte Käfig 1 unter Zuhilfenahme eines Distraktions-instrumentes in den ausgeräumten Zwischenwirbelraum eingeschoben. Dabei kann ein in die beiden Nuten 17 in der Frontwand 16 des Käfigs 1 eingeführtes Werkzeug als Manipulator dienen.
Der Käfig 1 kann entweder, wie in den Figuren 1 - 3 dargestellt als Halbimplantat ausgebildet sein, so dass zwei Implantate in den Zwischenwirbelraum eingeführt werden müssen. Es ist aber auch möglich zwei Halbimplantate einstückig auszubilden, so wie in den Fig. 4 - 7 dargestellt , so dass nur ein Implantat in den Zwischenwirbelraum eingeführt werden muss.

## Patentansprüche

1. Zwischenwirbel-Implantat mit einem einen Hohlraum (20) umschliessenden rahmenförmigen Käfig (1) mit einer perforierten Deck- und Grundfläche (11,12) als Knochenanlagefläche, zwei Seitenflächen (13,14), einer Frontwand (16) und einer Hinterwand (15), dadurch gekennzeichnet, dass
A) die Deck- und/oder Grundfläche (11,12) mit einer Mehrzahl von Perforationen (24) versehen ist, deren Gesamtfläche 40 % bis 55 % der Gesamtfläche der Deck- und/oder Grundfläche (11, 12) beträgt;
B) die Einzelfläche einer einzelnen Perforation (24) höchstens 20 % der Gesamtfläche der Deck- und/oder Grundfläche (11,12) beträgt;
C) das Verhältnis VH/VK zwischen Volumen VH des Hohlraums (20) und Gesamtvolumen VK des Käfigs (1) im Bereich von 0,11 bis 0,42, liegt; und
D) der Käfig (1) im wesentlichen keilförmig ausgebildet ist, mit gegen die Frontwand (16) hin divergierenden Deck- und Grundflächen (11,12).

2. Implantat nach Anspruch 1, dadurch gekennzeichnet, dass Deck- und Grundfläche (11,12) mit einer dreidimensionalen Strukturierung (18) versehen ist.

3. Implantat nach Anspruch 1 oder 2, dadurch gekennzeichnet, dass die im Randbereich der Deck- und/oder Grundfläche (11,12) angebrachten Perforationen (24) durchschnittlich kleiner sind als die im Zentralbereich der Deck- und/oder Grundfläche (11,12) angebrachten Perforationen (24).

4. Implantat nach einem der Ansprüche 1 bis 3, dadurch gekennzeichnet, dass Deck- und Grundfläche (11,12) nach aussen gewölbt ausgebildet sind.

5. Implantat nach einem der Ansprüche 1 bis 4, dadurch gekennzeichnet, dass der Durchmesser der Perforationen (24) höchstens 9,0 mm, vorzugsweise höchstens 5,0 mm beträgt.

6. Implantat nach einem der Ansprüche 1 bis 5, dadurch gekennzeichnet, dass die Seitenflächen (13,14) mit Perforationen (19) versehen sind, deren Gesamtfläche höchstens 40 % , vorzugsweise höchstens 30 % der Gesamtfläche der Seitenflächen (13,14) beträgt.

7. Implantat nach einem der Ansprüche 1 bis 6, dadurch gekennzeichnet, dass die Seitenflächen (13,14) mit Perforationen (19) versehen sind, deren Gesamtfläche mindestens 15 % , vorzugsweise mindestens 20 % der Gesamtfläche der Seitenflächen (13,14) beträgt.

8. Implantat nach einem der Ansprüche 2 bis 7, dadurch gekennzeichnet, dass die dreidimensionalen Strukturierung (18) eine strukturierte Hydroxylapatitbeschichtung ist.

9. Implantat nach einem der Ansprüche 2 bis 7, dadurch gekennzeichnet, dass die dreidimensionalen Strukturierung (18) eine strukturierte Beschichtung aus Titan, Titanlegierungen oder anderen körperverträglichen Metallen ist.

10. Implantat nach einem der Ansprüche 2 bis 7, dadurch gekennzeichnet, dass die dreidimensionalen Strukturierung (18) aus Zähnen besteht, vorzugsweise in regelmässiger Anordnung.

11. Implantat nach einem der Ansprüche 2 bis 10, dadurch gekennzeichnet, dass die dreidimensionale Strukturierung (18) ein Höhe von 0,5 - 2,0 mm, vorzugsweise von 1,0 - 1,5 mm aufweist.

12. Implantat nach einem der Ansprüche 1 bis 11, dadurch gekennzeichnet, dass die Frontwand (16) Mittel (17;27) zur Aufnahme eines Instrumentes aufweist, mit welchem der Käfig (1) manipuliert werden kann.

13. Implantat nach einem der Ansprüche 1 bis 12, dadurch gekennzeichnet, dass die Seitenflächen (13,14) Mittel (28) zur Aufnahme eines Instrumentes aufweisen, mit welchem der Käfig (1) manipuliert werden kann.

14. Implantat nach einem der Ansprüche 1 bis 13, dadurch gekennzeichnet, dass die Deck- und Grundfläche (11,12) einen freien Rand (32) ohne Strukturierung (18) aufweist.

15. Implantat nach einem der Ansprüche 1 bis 14, dadurch gekennzeichnet, dass die Perforationen (19) Langlochausnehmungen sind .

16. Implantat nach einem der Ansprüche 1 bis 15, dadurch gekennzeichnet, dass die Frontwand (16) mit Perforationen, vorzugsweise in Form von Längsausnehmungen, versehen ist.

17. Implantat nach einem der Ansprüche 1 bis 16, dadurch gekennzeichnet, dass der gesamte Käfig (1) mit Hydroxylapatit oder einem anderen bioaktiven Material beschichtet ist.

18. Implantat nach einem der Ansprüche 1 bis 17, dadurch gekennzeichnet, dass die Einzelfläche einer einzelnen Perforation (24) 5 - 15 %, vorzugsweise 8 - 13 % der Gesamtfläche der Deck- und/oder Grundfläche (11,12) beträgt.

19. Implantat nach einem der Ansprüche 1 bis 18, dadurch gekennzeichnet, dass das Verhältnis VH/VK zwischen Volumen des Hohlraums (20) VH und Gesamtvolumen VK des Käfigs (1) im Bereich von 0,17 bis 0,33, vorzugsweise von 0,21 bis 0,28 liegt.

20. Implantat nach einem der Ansprüche 1 - 19, dadurch gekennzeichnet, dass die Gesamtfläche der Perforationen (24) in der Deck- und/oder Grundfläche (11,12) 43 bis 51 %, vorzugsweise 45 - 49 % der Gesamtfläche der Deck- und/oder Grundfläche (11,12) beträgt.

21. Kombinationsimplantat mit zwei Zwischenwirbel-Implantaten nach einem der Ansprüche 1 - 20, dadurch gekennzeichnet, dass die beiden Zwischenwirbel-Implantate an ihren fehlenden Seitenflächen (14) einstückig miteinander verbunden sind.

22. Kombinationsimplantat nach Anspruch 21, dadurch gekennzeichnet, dass die kombinierte Frontwand (16) eine Langlochausnehmung aufweist.

## Claims

1. Intervertebral implant comprising a frame-like cage (1), which encloses a cavity (20) and which has a perforated cover and base faces (11;12) as bone-contacting surfaces, two lateral surfaces (13;14), a front wall (16) and a rear wall (15),
characterized in that
A) the cover and /or base face (11;12) is provided with a plurality of perforations (24), whose total area makes up 40% to 55% of the total area of the cover and/or base face (11;12);
B) each perforation (24) having an individual area amounting at most 20% of the total area of the cover and/or base face (11;12);
C) the ration VH/VK between the volume of the cavity (20) and the total volume of the cage (1) is in the range between 0,11 and 0,42; and
D) the cage (1) is essentially wedge-shaped whereby the cover and base faces (11;12) diverge toward the front wall (16).

2. Implant according to claim 1, characterized in that cover and base face (11;12) are provided with a three-dimensional structure (18).

3. Implant according to claim 1 or 2, characterized in that the perforations (24) affixed to the edge region of the cover and base face (11;12), on average, are smaller than the perforations (24) affixed in the central region of the cover and base face (11;12).

4. Implant according to one of the claims 1 to 3, characterized in that the cover and base face (11;12) are configured to bulge outward.

5. Implant according to one of the claims 1 to 4, characterized in that the perforations (24) have a maximum diameter of 9,0 mm, preferably maximum 5,0 mm.

6. Implant according to one of the claims 1 to 5, characterized in that the lateral surfaces (13;14) have perforations (19) whose total area is at most 40%, preferably 30% of the total area of the lateral surfaces (13;14).

7. Implant according to one of the claims 1 to 6, characterized in that the lateral surfaces (13;14) have perforations (19) whose total area is at least 15%, preferably at least 20% of the total area of the laterals surfaces (13;14).

8. Implant according to one of the claims 2 to 7, characterized in that the three-dimensional structure (18) is a structured hydroxyl-apatite coating.

9. Implant according to one of the claims 2 to 7, characterized in that the three-dimensional structure (18) is a structured coating of titanium, a titanium alloy or another body agreeable material.

10. Implant according to one of the claims 2 to 7, characterized in that the three-dimensional structure comprises teeth, preferably in regular arrangement.

11. Implant according to one of the claims 2 to 10, characterized in that the three-dimensional structure (18) has a height of 0,5 - 2,0 mm, preferably of 1,0 - 1,5 mm.

12. Implant according to one of the claims 1 to 11, characterized in that the front wall (16) has means (17;27) to receive an instrument for manipulating the cage (1).

13. Implant according to one of the claims 1 to 12, characterized in that the lateral surfaces (13;14) have means (28) to receive an instrument for manipulating the cage (1).

14. Implant according to one of the claims 1 to 13, characterized in that the cover face (11) and the base face (12) have an edge (32) which is free of three-dimensional structure (18).

15. Implant according to one of the claims 1 to 14, characterized in that the perforations (19) are longitudinal holes.

16. Implant according to one of the claims 1 to 15, characterized in that the front wall (16) is equipped with perforations, preferably in the form of longitudinal holes.

17. Implant according to one of the claims 1 to 16, characterized in that the entire cage (1) is coated with hydroxylapatite or another bioactive material.

18. Implant according to one of the claims 1 to 17, characterized in that each perforation (2) has an individual area which constitutes 5 - 15%, preferably 8 - 13% of the total area of the cover and/or base face (11;12).

19. Implant according to one of the claims 1 to 18, characterized in that the ration VH/VK between the volume of the cavity (20) and the total volume of the cage (1) ranges between 0,17 and 0,33, preferably between 0,21 and 0,28.

20. Implant according to one of the claims 1 to 19, characterized in that the total area of the perforations (24) in the cover and/or base face (11;12) amounts to 43 to 51%, preferably 45 - 49% of the total area of the cover and/or base face (11;12).

21. Combined implant with two intervertebral implants according to one of the claims 1 to 20, characterized in that the two intervertebral implants are joined integrally together at their missing lateral surface (14).

22. Combined implant according to claim 21, characterized in that the combined front wall (16) has a longitudinal hole recess.

## Revendications

1. Implant intervertébral présentant une cage (1) en forme de cadre entourant un espace vide (20) et dotée d'une surface de chapeau et d'une surface de base perforées (11, 12) qui servent de surfaces de pose sur l'os, de deux surfaces latérales (13, 14), d'une paroi frontale (16) et d'une paroi arrière (15), caractérisé en ce que
A) la surface de chapeau et/ou la surface de base (11, 12) sont dotées d'une pluralité de perforations (24) dont la superficie totale représente 49 % à 55 % de la superficie totale de la surface de chapeau et/ou de la surface de base (11, 12);
B) la superficie individuelle d'une perforation individuelle (24) représente au plus 20 % de la superficie totale de la surface de chapeau et/ou de la surface de base (11, 12);
C) le rapport VH/VK entre le volume VH de l'espace vide (20) et le volume total VK de la cage (1) est compris dans la plage de 0,11 à 0,42 ; et
D) la cage (1) est configurée essentiellement en biseau, avec une surface de chapeau et une surface de base (11, 12) qui divergent en direction de la paroi frontale (16).

2. Implant selon la revendication 1, caractérisé en ce que la surface de chapeau et la surface de base (11, 12) sont dotées d'une structuration tridimensionnelle (18).

3. Implant selon la revendication 1 ou 2, caractérisé en ce que les perforations (24) ménagées dans la zone de bordure de la surface de chapeau et/ou de la surface de base (11, 12) sont en moyenne plus petites que les perforations (24) ménagées dans la région centrale de la surface de chapeau et/ou de la surface de base (11, 12).

4. Implant selon l'une des revendications 1 à 3, caractérisé en ce que la surface de chapeau et la surface de base (11, 12) sont configurées avec une courbure tournée vers l'extérieur.

5. Implant selon l'une des revendications 1 à 4, caractérisé en ce que le diamètre des perforations (24) vaut au plus 9,0 mm, et de préférence au plus 5,0 mm.

6. Implant selon l'une des revendications 1 à 5, caractérisé en ce que les surfaces latérales (13, 14) sont dotées de perforations (19) dont la superficie totale représente au plus 40 %, de préférence au plus 30 % de la superficie totale des surfaces latérales (13, 14).

7. Implant selon l'une des revendications 1 à 6, caractérisé en ce que les surfaces latérales (13, 14) sont dotées de perforations (19) dont la superficie représente au moins 15 %, de préférence au moins 20 % de la superficie totale des surfaces latérales (13, 14).

8. Implant selon l'une des revendications 1 à 7, caractérisé en ce que la structuration tridimensionnelle (18) est un revêtement d'hydroxyapatite structuré.

9. Implant selon l'une des revendications 2 à 7, caractérisé en ce que la structuration tridimensionnelle (18) est un revêtement structuré de titane, d'alliages de titane ou d'autres métaux compatibles avec le corps.

10. Implant selon l'une des revendications 2 à 7, caractérisé en ce que la structuration tridimensionnelle (18) est constituée de dents, de préférence agencées de manière régulière.

11. Implant selon l'une des revendications 2 à 10, caractérisé en ce que la structuration tridimensionnelle (18) présente une hauteur de 0,5 à 2,0 mm, de préférence de 1,0 à 1,5 mm.

12. Implant selon l'une des revendications 1 à 11, caractérisé en ce que la paroi frontale (16) présente des moyens (17;27) pour la réception d'un instrument qui permet de manipuler la cage (1).

13. Implant selon l'une des revendications 1 à 12, caractérisé en ce que les surfaces latérales (13, 14) présentent des moyens (28) pour la réception d'un instrument qui permet de manipuler la cage (1).

14. Implant selon l'une des revendications 1 à 13, caractérisé en ce que la surface de chapeau et la surface de base (11, 12) présentent un bord libre (32) sans structuration (18).

15. Implant selon l'une des revendications 1 à 14, caractérisé en ce que les perforations (19) sont des découpes en trous oblongs.

16. Implant selon l'une des revendications 1 à 15, caractérisé en ce que la paroi frontale (16) est dotée de perforations qui présentent de préférence la forme de découpes allongées.

17. Implant selon l'une des revendications 1 à 16, caractérisé en ce que l'ensemble de la cage (1) est revêtue d'hydroxyapatite ou d'un autre matériau bioactif.

18. Implant selon l'une des revendications 1 à 17, caractérisé en ce que la superficie individuelle d'une perforation individuelle (24) représente de 5 à 15 %, de préférence de 8 à 13 % de la superficie totale de la surface de chapeau et/ou de la surface de base (11, 12).

19. Implant selon l'une des revendications 1 à 18, caractérisé en ce que le rapport VH/VK entre le volume de l'espace vide (20) VH et le volume total VK de la cage (1) est compris dans la plage de 0,17 à 0,33, de préférence de 0,21 à 0,28.

20. Implant selon l'une des revendications 1 à 19, caractérisé en ce que la superficie totale des perforations (24) ménagée dans la surface de chapeau et/ou dans la surface de base (11, 12) représente de 43 à 51 %, de préférence de 45 à 49 % de la superficie totale de la surface de chapeau et/ou de la surface de base (11, 12).

21. Implant combiné présentant deux implants intervertébraux selon l'une des revendications 1 à 20, caractérisé en ce que les deux implants intervertébraux sont reliés l'un à l'autre d'un seul tenant par leur surface latérale (14) manquante.

22. Implant combiné selon la revendication 21, caractérisé en ce que la partie frontale combinée (16) présente un évidemment en forme de trou oblong.
